# EUROPEAN PATENT APPLICATION

(11) **EP 1 393 740 A1**
(43) Date of publication of application: **03.03.2004**
(21) Application number: 02769564.2
(22) Date of filing: 10.05.2002
(51) Int. Cl.: A61K 38/17, A61K 38/00, A61P 25/00, A61P 25/16, A61P 25/28, A61P 25/14, A61P 25/08

(54) **NOVEL REMEDIES FOR NEURODEGENERATIVE DISEASE**

(30) Priority: 11.05.2001 JP 2001141462
(71) Applicant: Juridical Foundation, The Chemo-Sero-Therapeutic Research Institute, Kumamoto-shi, Kumamoto 860-8568 (JP); Hisamitsu Pharmaceutical Co., Inc., Tosu-shi, Saga 841-8686 (JP)
(72) Inventor: HIRASHIMA, Masaki, KIKUCHI RESEARCH CENTER, Kikuchi-gun, Kumamoto 869-1298 (JP); NARUSE, Takeshi, KIKUCHI RESEARCH CENTER, Kikuchi-gun, Kumamoto 869-1298 (JP); MAEDA, Hiroaki, KIKUCHI RESEARCH CENTER, Kikuchi-gun, Kumamoto 869-1298 (JP); NOZAKI, Chikateru, Kikuchi Research Center, Kikuchi-gun, Kumamoto 869-1298 (JP); GOTO, Takeshi, c/o HISAMITSU PHARMA. CO. INC., Tsukuba-shi, Ibaraki 305-0856 (JP); AKIYAMA, Katsuhiko, c/o HISAMITSU PHARMA. CO. INC., Tsukuba-shi, Ibaraki 305-0856 (JP); FUKUSHIMA, Hidenao, c/o HISAMITSU PHARMA. CO. INC., Tsukuba-shi, Ibaraki 305-0856 (JP)
(74) Representative: VOSSIUS & PARTNER
(86) International application number: PCT/JP2002/004558
(87) International publication number: WO 2002/092121

(57) **Abstract**

A medicament for treating neurodegenerative diseases, comprising as an active ingredient selenoprotein P and/or a peptide fragment or a series of peptide fragments derived from the C-terminal of selenoprotein P. An excellent medicament for treating neurodegenerative diseases, especially suitable for treating neurodegenerative diseases with ataxia as a principal symptom is provided.

SEQUENCE LISTING
<110> JURIDICAL FOUNDATION THE CHEMO-SERO-THERAPEUTIC RESEARCH INSTITUTE
HISAMITSU SEIYAKU K.K.

<120> NOVEL MEDICAMENT FOR TREATING NEURODEGENERATIVE DISEASES

<130> 663196

<150> JP 2001-141462
<151> 2001-05-11

<160> 15

<210> 1
<211> 14
<212> PRT
<213> Human plasma

<220>
<223> Xaa represents selenocysteine

<400> 1
Arg Ser Xaa Cys Cys His Cys Arg His Leu Ile Phe Glu Lys
1               5                   10

<210> 2
<211> 103
<212> PRT

<213> Human plasma

<220>
<223> Fragment of selenoprotein P
<223> Xaa represents selenocysteine

<400> 2
Lys Arg Cys Ile Asn Gln Leu Leu Cys Lys Leu Pro Thr Asp Ser Glu
260 264 269 274
Leu Ala Pro Arg Ser Xaa Cys Cys His Cys Arg His Leu Ile Phe Glu
279 284 289
Lys Thr Gly Ser Ala Ile Thr Xaa Gln Cys Lys Glu Asn Leu Pro Ser
294 299 304
Leu Cys Ser Xaa Gln Gly Leu Arg Ala Glu Glu Asn Ile Thr Glu Ser
309 314 319
Cys Gln Xaa Arg Leu Pro Pro Ala Ala Xaa Gln Ile Ser Gln Gln Leu
324 329 334 339
Ile Pro Thr Glu Ala Ser Ala Ser Xaa Arg Xaa Lys Asn Gln Ala Lys
344 349 354
Lys Xaa Glu Xaa Pro Ser Asn
359

<210> 3
<211> 33
<212> PRT
<213> Human plasma

<220>

<223> Xaa represents selenocysteine

<400> 3

Lys Arg Cys Ile Asn Gln Leu Leu Cys Lys Leu Pro Thr Asp Ser Glu

1               5                   10                  15

Leu Ala Pro Arg Ser Xaa Cys Cys His Cys Arg His Leu Ile Phe Glu

            20                  25                  30

Lys

<210> 4

<211> 21

<212> PRT

<213> Human plasma

<400> 4

Lys Arg Cys Ile Asn Gln Leu Leu Cys Lys Leu Pro Thr Asp Ser Glu

1               5                   10                  15

Leu Ala Pro Arg Ser

            20

<210> 5

<211> 14

<212> PRT

<213> Human plasma

<400> 5

Arg Ser Ser Cys Cys His Cys Arg His Leu Ile Phe Glu Lys

1                   5                   10

<210> 6

<211> 14

<212> PRT

<213> Human plasma

<220>

<223> Xaa represents selenocysteine

<400> 6

Arg Ser Xaa Ser Cys His Cys Arg His Leu Ile Phe Glu Lys

1                   5                   10

<210> 7

<211> 14

<212> PRT

<213> Human plasma

<220>

<223> Xaa represents selenocysteine

<400> 7

Arg Ser Xaa Ser Ser His Cys Arg His Leu Ile Phe Glu Lys

1                   5                   10

<210> 8

<211> 14

<212> PRT

<213> Human plasma

<220>

<223> Xaa represents selenocysteine

<400> 8

Arg Ser Xaa Ser Ser His Ser Arg His Leu Ile Phe Glu Lys

1               5                   10

<210> 9

<211> 4

<212> PRT

<213> Human plasma

<220>

<223> Xaa represents selenocysteine

<400> 9

Arg Ser Xaa Ser

1

<210> 10

<211> 10

<212> PRT

<213> Human plasma

<220>
<223> Xaa represents selenocysteine

<400> 10
Thr Gly Ser Ala Ile Thr Xaa Gln Ser Lys
1               5                   10

<210> 11
<211> 13
<212> PRT
<213> Human plasma

<220>
<223> Xaa represents selenocysteine

<400> 11
Glu Asn Leu Pro Ser Leu Ser Ser Xaa Gln Gly Leu Arg
1               5                   10

<210> 12
<211> 12
<212> PRT
<213> Human plasma

<220>

<223> Xaa represents selenocysteine

<400> 12

Ala Glu Glu Asn Ile Thr Glu Ser Ser Gln Xaa Arg

1               5                   10

<210> 13

<211> 11

<212> PRT

<213> Human plasma

<220>

<223> Xaa represents selenocysteine

<400> 13

Leu Ile Pro Thr Glu Ala Ser Ala Ser Xaa Arg

1               5                   10

<210> 14

<211> 8

<212> PRT

<213> Human plasma

<220>

<223> Xaa represents selenocysteine

<400> 14

Lys Asn Gln Ala Lys Lys Xaa Glu
1               5

<210> 15
<211> 4
<212> PRT
<213> Human plasma

<220>
<223> Xaa represents selenocysteine

<400> 15
Xaa Pro Ser Asn
1

<210> 16
<211> 15
<212> PRT
<213> Human plasma

<220>
<223> Xaa represents selenocysteine

<400> 16
Lys Glu Phe Ile Leu His Arg Ser His Ser Ser Xaa Ser Arg
1               5                   10                  15

<210> 17

<211> 11

<212> PRT

<213> Human plasma

<220>

<223> Xaa represents selenocysteine

<400> 17

Leu Pro Pro Ala Ala Xaa Gln Ile Ser Gln Gln

1               5                   10

## Description

### TECHNICAL FIELD OF THE INVENTION

The present invention relates to a novel use of plasma proteins, belonging to the field of medical drugs. Specifically, the present invention relates to a medicament for treating neurodegenerative diseases causing ataxia. More specifically, the present invention relates to a medicament for treating neurodegenerative diseases comprising as an active ingredient selenoprotein P, one of plasma proteins, preferably a peptide fragment or a series of peptide fragments derived from the C-terminal of selenoprotein P.

### BACKGROUND OF THE INVENTION

Neurodegenerative diseases are known to cause decrease in motor function (ataxia) etc. Neurodegenerative diseases include, for instance, Alzheimer type senile dementia, Pick disease, Huntington chorea, Parkinson disease, spinocerebellar degeneration, progressive supranuclear palsy, intractable epilepsy, and the like.

Central nervous system disorders leading to ataxia is classified according to the regions suffered into cerebral (frontal lobe), cerebellar, vestibular (labyrinth), and spinal ataxia. Cerebral ataxia is caused by disorder in cerebral cortex, especially the frontal lobe, and can be observed in case of cerebrovascular lesion, cerebral atrophy, trauma, tumor, Pick disease, and chronic subdural hematoma. It exhibits atactic abasia and decrease in mental function. Cerebellar ataxia is a significant symptom associated with, for instance, cerebellar disorders such as cerebellar tumor, vascular disorders, degenerative disorders, cerebellar atrophy, or deformity. Lesions in the vermis induce trunk ataxia, exhibits astasia-abasia, and gluteus maximus gait, yields difficulty in maintaining posture and position with disorder in balance.

On the other hand, disorders in the cerebellar hemisphere exhibit abnormality of tonus in limb muscles and decrease in myotony and are accompanied by maldispositional gait towards the affected lateral direction, incoordination, wrong indication in finger-finger test or finger-nose test, dysmetria, Holmes-Stewart phenomenon, as well as intention tremor and cerebellar speech (scanning, explosive). Vestibular (labyrinth) ataxia is caused by vestibular malfunctions and most of its cause is supposed to be the presence of, or sequela from, otological disorders in the internal ear, including, for instance, Ménière disease, sudden deafness, disorders in the balance-related nerves due to drug poisoning such as streptomycin or kanamycin, trauma, syphilis, acoustic trauma hearing loss, otosclerosis, and otitis interna (and its sequela). In case of spinal ataxia, also called ataxia of posterior funiculus, disorders in posterior column of spinal cord lead to disorders in bathyesthesia, i.e. positional sensibility, articular sensibility and sensibility of grasp, resulting in ataxia. It is markedly observed in Friedreich's ataxia, subacute combined degeneration of spinal cord, locomotor ataxia, and the like.

For example, spinocerebellar degeneration (SCD) with a principal symptom of ataxia is known which is a generic name for neurodegenerative diseases with unknown causes. Clinically, its principal symptom is cerebellar or posterior funicular ataxia. Its progression is such that symptoms of ataxia gradually onset and slowly progress. In some cases, SCD only exhibits ataxia, trunk ataxia or incoordination. However, SCD sometimes displays other symptoms, including cerebellar mogilalia, extrapyramidal signs, in particular parkinsonism such as muscular rigidity and akinesia, pyramid sign such as an accelerated or even abnormal tendon reflex, nystagmus or involuntary motion (peripheral nervous symptoms), autonomous symptoms such as orthostatic hypotension or dysuria, and less frequently intelligence disorder. Accordingly, SCD is considered to be typical neurodegenerative diseases with ataxia.

SCD is classified according to the principal lesions into (1) cerebellar type, including Holmes type with lesions in the cerebellar cortex; (2) spinocerebellar type, including Menzel type olivopontocerebellar atrophy; and (3) spinal cord type, including Friedreich's ataxia or hereditary spastic ataxia. According to statistics carried out by the Ataxia Search Group, occurrence of SCD patients in Japan in 1990 was about 7 to 10 cases per 1 x 10⁵ people, among which about 60% comprised non-hereditary cases whereas 40% hereditary cases. Among the non-hereditary cases, most was olivopontocerebellar atrophy, which thus most frequently occurs among total spinocerebellar degeneration cases. On the other hand, as for the hereditary cases, many of them were hitherto diagnosed as Menzel type hereditary ataxia. However, with the progress in genetic diagnosis in recent years, it was found that Machado-Joseph disease (MJD) was the most frequent. On the other hand, causes of the non-hereditary types such as olivopontocerebellar atrophy remain completely unknown. Thus, in spite of extensive study to elucidate the causes from the genetic level, the reason why the cerebellum or the neurocytes associated with the cerebellum are selectively lead to death is still to be elucidated.

### DISCLOSURE OF THE INVENTION

The only medicament hitherto known for treating ataxia symptoms of spinocerebellar degeneration is preparations of thyrotropin-releasing hormone (TRH) tartarate for intravenous or intramuscular administration (Hirtonin). Although the mechanism of activity is scarcely known, it sometimes ameliorates ataxia, articulation disorder, motion speed, and the like, in case of early stage or in mild cases. However, duration of the efficacy is as short as 1 hour and hence the efficacy of remedy is not so much appreciated. Besides, this drug has an activity to promote secretion of thyrotropin (TSH), thus raising concern for side effects. Recently, oral preparations of TRH-T derivatives (Ceredist) have been developed. This new drug however merely exhibits duration of the efficacy that is prolonged thrice as compared to the conventional one as evidenced in the experiment using Rolling Mouse Nagoya in which intraperitoneal administration of the TRH derivative showed duration of about 3 hours as compared to about 1 hour in case of TRH. As such, a basic administration pattern of daily dosage for 2 to 3 weeks followed by ceasing of administration for 2 to 3 weeks is not significantly altered. Other than these drugs, symptomatic treatment has predominantly been used, such as a medicament for Parkinson disease to treat parkinsonism, e.g. tremor in hands, or a medicament for autonomous regulation to treat autonomous symptoms, e.g. orthostatic hypotension. The circumstances are similar in various diseases with ataxia as mentioned above and hence little medicament is known for effectively ameliorating symptoms of ataxia. Accordingly, there is a desire for developing a novel medicament for treating with high efficacy neurodegenerative diseases exhibiting ataxia as a principal symptom.

Under the circumstances, the present inventors have found that selenoprotein P, a protein derived from blood components, more preferably a peptide fragment from the C-terminal of selenoprotein P, exhibits a cell death-inhibitory activity, which hitherto has not been reported, and have filed a patent application (PCT/JP99/06322) for this finding. The present inventors further investigated for providing a novel medicament for ameliorating neurodegenerative diseases. As a result, selenoprotein P or a peptide fragment or a series of peptide fragments derived from the C-terminal of selenoprotein P surprisingly proved to be efficacious as a medicament for treating neurodegenerative diseases in humans or other animals as demonstrated in animal models which received in vivo administration thereof. Based on this finding, the present inventors have thus completed the present invention.

That is, the present invention relates to a medicament for treating neurodegenerative diseases comprising as an active ingredient selenoprotein P and/or a peptide fragment or a series of peptide fragments derived from the C-terminal of selenoprotein P.

In a preferable embodiment of the present invention, the peptide fragment or a series of the peptide fragments from the C-terminal of selenoprotein P is one having the amino acid sequence from 260th to 362nd amino acids from the C-terminal of selenoprotein P, or having said amino acid sequence with one or several amino acid residues therein being deleted, substituted or added, or having a partial sequence of either of the above amino acid sequences, or having any of the above amino acid sequences as a part of a whole sequence, having a cytotoxicity-inhibitory activity.

In a more preferable embodiment of the present invention, the peptide fragment or a series of the peptide fragments from the C-terminal of selenoprotein P has the amino acid sequences of the formula: Arg Ser Xaa Cys Cys His Cys Arg His Leu Ile Phe Glu Lys (SEQ ID NO: 1) wherein Xaa represents selenocysteine, or said amino acid sequences with one or several amino acid residues therein being deleted, substituted or added, or a partial sequence of either of the above amino acid sequences, or an amino acid sequence comprising as a part any of the above amino acid sequences.

### BRIEF DESCRIPTION OF THE DRAWINGS

Fig. 1 shows an effect of selenoprotein P to ameliorate a stumbling index on the 1st week of administration of the protein in Rolling Mouse Nagoya.
Fig. 2 shows an effect of selenoprotein P to ameliorate a stumbling index on the 2nd week of administration of the protein in Rolling Mouse Nagoya.
Fig. 3 shows an effect of selenoprotein P to ameliorate a stumbling index on the 3rd week of administration of the protein in Rolling Mouse Nagoya.

### BEST MODE FOR CARRYING OUT THE INVENTION

Selenoprotein P was identified in 1977 as a selenium-containing protein other than glutathione-peroxidase. In 1982, it was revealed that selenium was incorporated into said protein in the form of selenocysteine. Moreover, in 1991, a full-length amino acid sequence of selenoprotein P was determined by cloning selenoprotein P cDNA and, as a result, possibility that said protein contains at most ten selenocysteine residues was demonstrated (Hill K. E. and Burk R. F., Biomed. Environ. Sci., 10, p. 198-208 (1997)). Little was known about the function of selenoprotein P. However, it has recently been demonstrated that selenoprotein P exhibits an activity to reduce phospholipid hydroperoxide or peroxynitrite in vitro and acts as a survival promoting factor of neurocytes.

As demonstrated in the Examples hereinbelow, selenoprotein P proved to have an activity to lower a stumble index (i.e. frequency of stumbling/voluntary motion) in the experiment where Rolling Mouse Nagoya, model mice of spinocerebellar degeneration, one of neurodegenerative diseases, received intraperitoneal administration of selenoprotein P, and hence to ameliorate ataxia. It was thus demonstrated that selenoprotein P had an activity to treat neurodegenerative diseases with ataxia as a principal symptom.

The present invention relates to a novel pharmaceutical efficacy of selenoprotein P based on the new findings as mentioned above and an active ingredient of a medicament for treating neurodegenerative diseases of the present invention is selenoprotein P. More specifically, selenocysteine, a selenium-containing amino acid, contained in selenoprotein P is thought to be responsible for amelioration of ataxia. The present inventors have found that a peptide fragment derived from the C-terminal of selenoprotein P, a protein from blood components, exhibited a cell death-inhibitory activity, which hitherto has not been reported, and filed a patent application. Selenocysteine contained in selenoprotein P is apparently involved in this activity. Hence, a protein and/or a series of peptides that contains selenocysteine and has a cell death-inhibitory activity can be a candidate of a medicament for treating neurodegenerative diseases.

Selenium per se, as involved in the present invention, is one of essential trace elements and it is known that deficiency thereof induces a serious deficiency disease accompanied by, for instance, cardiomyopathy. It is also demonstrated that selenium is essential for survival, maintenance of life or growth of cells as can be seen from that addition of sodium selenite to culture medium is indispensable during serum-free culture. However, as will be understood from the fact that selenium compounds are designated as poisonous substance, a difference between effective and toxic amounts, i.e. a safety range of concentration, is small and hence selenium compounds used in an excess amount may be toxic to cells to induce unfavorably cell death. Acute toxic symptoms of selenium include, for example, pale face, neurological symptoms, dermatitis, and gastrointestinal disorders. It is also known that selenocystine, a dimer of selenocysteine, exhibits fairly strong toxicity when added alone to cell culture.

On the contrary, no strong toxicity was observed in selenoprotein P or a peptide fragment derived from the C-terminal of selenoprotein P according to the present invention in spite of the presence of 9 to 10 selenocysteines therein. From this, selenoprotein P with the pharmaceutical efficacy according to the present invention is characteristic in that it not only contains selenocysteine but also possesses reduced toxicity. In fact, selenocysteine-containing synthetic peptides comprising 4 to 14 amino acid residues from the amino acid sequence of selenoprotein P exhibited no toxicity at a concentration where selenocystine exhibited toxicity. Thus, a peptide or a series of peptides of the present invention allows for providing selenium compounds that not only have reduced toxicity but also exhibit an unexpected activity to ameliorate ataxia.

Selenoprotein P as used herein includes any selenoprotein P in any molecular type without any restriction as far as it exhibits a desired activity to ameliorate ataxia, including selenoprotein P as an intact molecule or in any of other various molecule types. Among these, preferred is a peptide fragment or a series of the peptide fragments from the C-terminal of selenoprotein P. Most preferred is the peptide fragment or a series of the peptide fragments from the C-terminal of selenoprotein P that has the amino acid sequence consisting of 103 amino acid residues from the C-terminal of selenoprotein P (260th to 362nd amino acids), or said amino acid sequence with one or several amino acid residues therein being deleted, substituted or added, or a partial sequence of either of the above amino acid sequences, or an amino acid sequence comprising as a part any of the above amino acid sequences. For amino acid substitution, Cys is preferably replaced with Ser.

The most preferable peptide according to the present invention is one having the amino acid sequences of the formula: Arg Ser Xaa Cys Cys His Cys Arg His Leu Ile Phe Glu Lys (SEQ ID NO: 1) wherein Xaa represents selenocysteine, or said amino acid sequences with one or several amino acid residues therein being deleted, substituted or added, or a partial sequence of either of the above amino acid sequences, or an amino acid sequence comprising as a part any of the above amino acid sequences.

The term "a series of the peptide fragments" as used herein refers to a group of peptide fragments with different minute structures due to presence or absence of glycosylation, difference in electric charge, diversity in fragmentation, etc., each of the peptide fragments comprising about 4 to about 14 amino acid residues derived from the amino acid sequence of selenoprotein P, having at least one selenocysteine, or having said amino acid sequence with one or several amino acid residues therein being deleted, substituted or added. That is, selenoprotein P and a series of the peptide fragments according to the present invention includes any molecules that are derived from the amino acid sequence of selenoprotein P and have an activity to ameliorate ataxia, including selenoprotein P as an intact molecule as well as peptide fragments from the C-terminal of selenoprotein P. The peptide fragments of the present invention may be prepared by the conventional methods using a peptide synthesizer. Alternatively, chemical compounds may also be designed by using the peptide fragments of the present invention as a lead substance.

Selenoprotein P or the peptide fragment or a series of the peptide fragments derived from said protein for use in the present invention may be prepared by any process known in the art, for example, by isolation from human blood, or by the genetic recombination technique. Selenoprotein P or the peptide fragment or a series of the peptide fragments derived from said protein for use in the present invention as an active ingredient of a medicament for treating neurodegenerative diseases is rather stable to heat, a denaturing agent, a broad range of pH or protease in blood as compared to common enzymes. Thus, for purification and identification thereof, a wide variety of fractionation procedures may be applicable, including, for example, fractionations with applicable various carriers such as various chromatographic procedures such as heparin chromatography, cation exchange chromatography, anion exchange chromatography, hydrophobic chromatography, gel filtration chromatography, reverse phase chromatography, hydroxyapatite chromatography, or affinity chromatography with a column bound with an antibody, using plasma as a starting material. In addition to these, other various fractionations may also be applicable such as ammonium sulfate precipitation, molecular size fractionation with membrane, isoelectric focusing, electrophoretic fractionation, etc. A various combination of these fractionations may suitably be used to effectively fractionate selenoprotein P or the peptide fragment or a series of the peptide fragments derived from said protein. In a preferable embodiment, the peptide fragment or a series of the peptide fragments can be purified by conducting heparin chromatography, ammonium sulfate precipitation, anion exchange chromatography, cation exchange chromatography, hydrophobic chromatography, heparin chromatography, gel filtration chromatography, reverse phase chromatography and anion exchange chromatography in this order.

Selenoprotein P or the peptide fragment or a series of the peptide fragments derived from said protein for use in the present invention as an active ingredient of a medicament for treating neurodegenerative diseases can also be purified by affinity chromatography using a suitable carrier to which an appropriate antibody against said protein is bound. An example of preferable combination of such affinity chromatography with cation exchange chromatography mentioned above is shown in Preparation 1.

According to the present invention, selenoprotein P or the peptide fragment or a series of the peptide fragments derived from said protein as an active ingredient may be combined with a suitable known excipient to produce a medicament for treating neurodegenerative diseases. An effective dose of the medicament for treating neurodegenerative diseases of the present invention may vary depending upon ages of subject, symptoms, severity, etc. and ultimately upon discretion of a physician. A pharmaceutical efficacy does not depend upon a route of administration but subcutaneous, intradermal, or intraperitoneal administration, or bolus administration within blood vessels or intravenous drip infusion is much preferred. It is also possible to administer orally or transdermally in case of peptides with a low molecular weight.

A medicament for treating neurodegenerative diseases of the present invention may suitably be applied in general to neurodegenerative diseases, most effective for those with ataxia (decrease in motor function) as a principal symptom. A medicament for treating neurodegenerative diseases of the present invention comprising as an active ingredient selenoprotein P or a peptide or a series of peptides derived from said protein may be administered alone or in combination with other medical drug where synergetic effect may be expected.

The present invention is explained in more detail by means of the following Preparation and Examples which are not intended to restrict a scope of the present invention in any sense. Reagents used in the following Preparation and Examples were obtained from Wako Pure Chemical Industries, Ltd., TAKARA SHUZO CO., Ltd., Toyobo, and New England BioLabs.

### Preparation 1

### (Purification of Selenoprotein P Fragment Using Anti-Selenoprotein P Fragment Antibody-Bound Carrier (Anti-SeP Antibody Column))

As described below, selenoprotein P and selenoprotein P fragments were purified from plasma based on the cell death-inhibitory activity of selenoprotein P.

Heparin Sepharose-binding fraction from plasma was precipitated with 2 M ammonium sulfate. The precipitate was dissolved in more than 5 volumes of 20 mM Tris buffer, pH 8.0. Selenoprotein P present in this solution was adsorbed to a carrier to which an antibody against selenoprotein P fragment was bound (anti-SeP antibody column) and the carrier was washed with PBS. Selenoprotein P was eluted with 20 mM citrate buffer containing 4 M urea and was adsorbed to a cation exchanger (Macroprep High S: BioRad) equilibrated with 20 mM citrate buffer. Then, gradient elution was performed with a salt concentration of sodium chloride and a fraction of selenoprotein P fragments having the cell death-inhibitory activity was recovered. At this stage, a full-length selenoprotein P could also be obtained but with a cell death-inhibitory activity per proteins being much lower than that of the fragment thereof. According to the procedures as described herein, purification may be carried out in a short time and hence selenoprotein P fragments with higher cell death-inhibitory activity per proteins could be obtained. The fragments obtained at this stage were a fraction of a mixture containing various molecular species with varied sizes depending on the presence or absence of glycosylation, intermolecular bonding, or inner cleavage, etc. They were a group of selenoprotein P fragments that showed a size ranging from 10 to 30 kDa in electrophoresis under non-reductive condition.

### Preparation 2

### (Peptide Synthesis)

Selenocysteine was protected with Fmoc (9-Fluorenylmethoxycarbonyl) or MBzl (p-Methoxybenzyl). With the protected selenocysteine, desired peptides were synthesized by the Fmoc technique with a peptide synthesizer. Then, the peptides were deprotected and purified by reverse phase HPLC.

The present inventors confirmed that a peptide having the amino acid sequence: Lys Arg Cys Ile Asn Gln Leu Leu Cys Lys Leu Pro Thr Asp Ser Glu Leu Ala Pro Arg Ser Xaa Cys Cys His Cys Arg His Leu Ile Phe Glu Lys (SEQ ID NO: 3) wherein Xaa represents selenocysteine had the cell death-inhibiting activity, which peptide was purified under reduced condition from selenoprotein P fragment having the amino acid sequence: 260Lys Arg Cys Ile Asn Gln Leu Leu Cys Lys Leu Pro Thr Asp Ser Glu Leu Ala Pro Arg Ser Xaa Cys Cys His Cys Arg His Leu Ile Phe Glu Lys Thr Gly Ser Ala Ile Thr Xaa Gln Cys Lys Glu Asn Leu Pro Ser Leu Cys Ser Xaa Gln Gly Leu Arg Ala Glu Glu Asn Ile Thr Glu Ser Cys Gln Xaa Arg Leu Pro Pro Ala Ala Xaa Gln Ile Ser Gln Gln Leu Ile Pro Thr Glu Ala Ser Ala Ser Xaa Arg Xaa Lys Asn Gln Ala Lys Lys Xaa Glu Xaa Pro Ser Asn362 (SEQ ID NO: 2) wherein Xaa is as defined above, having the cell death-inhibiting activity. Based on this, peptide fragments having the amino acid sequences as described below were synthesized.

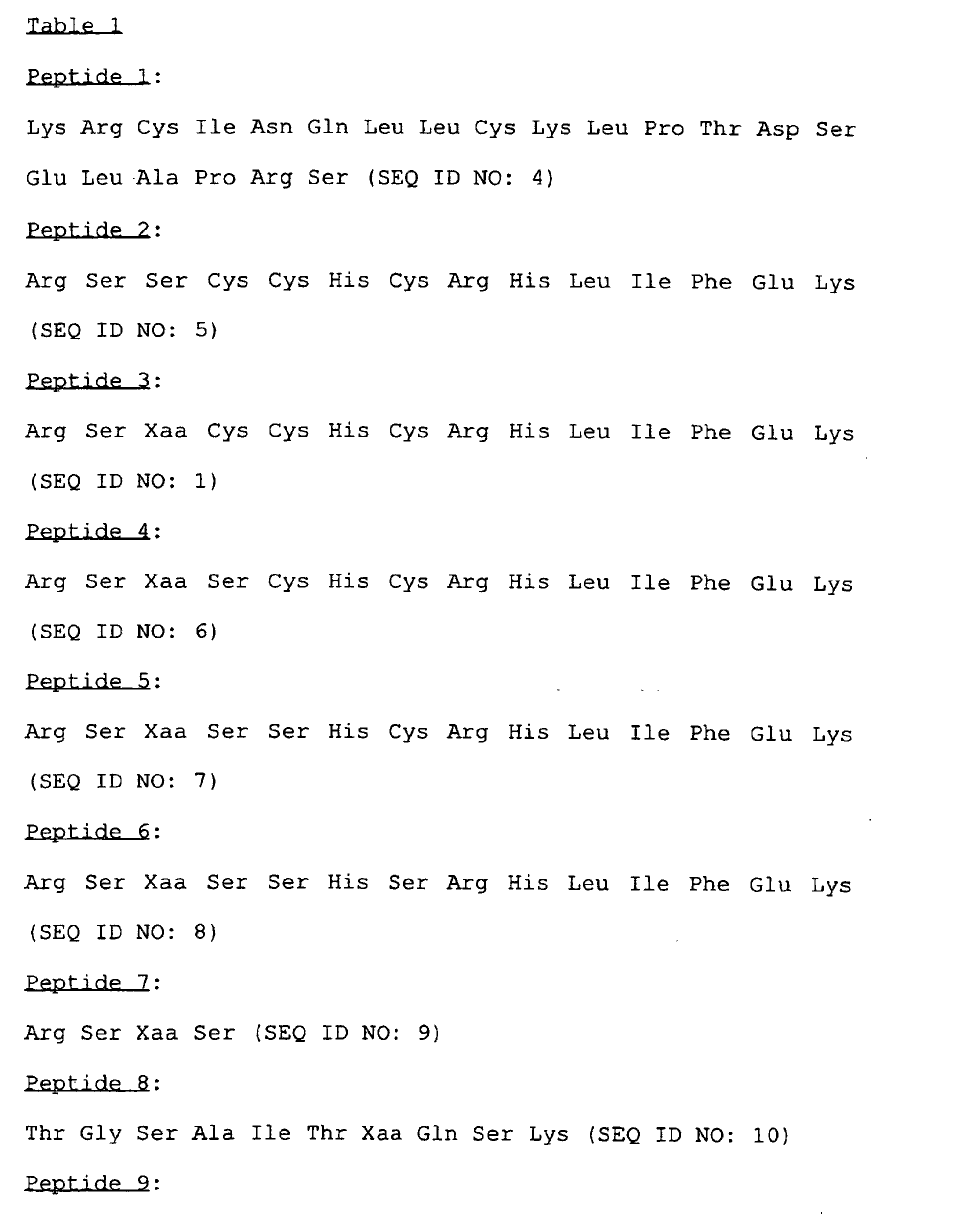

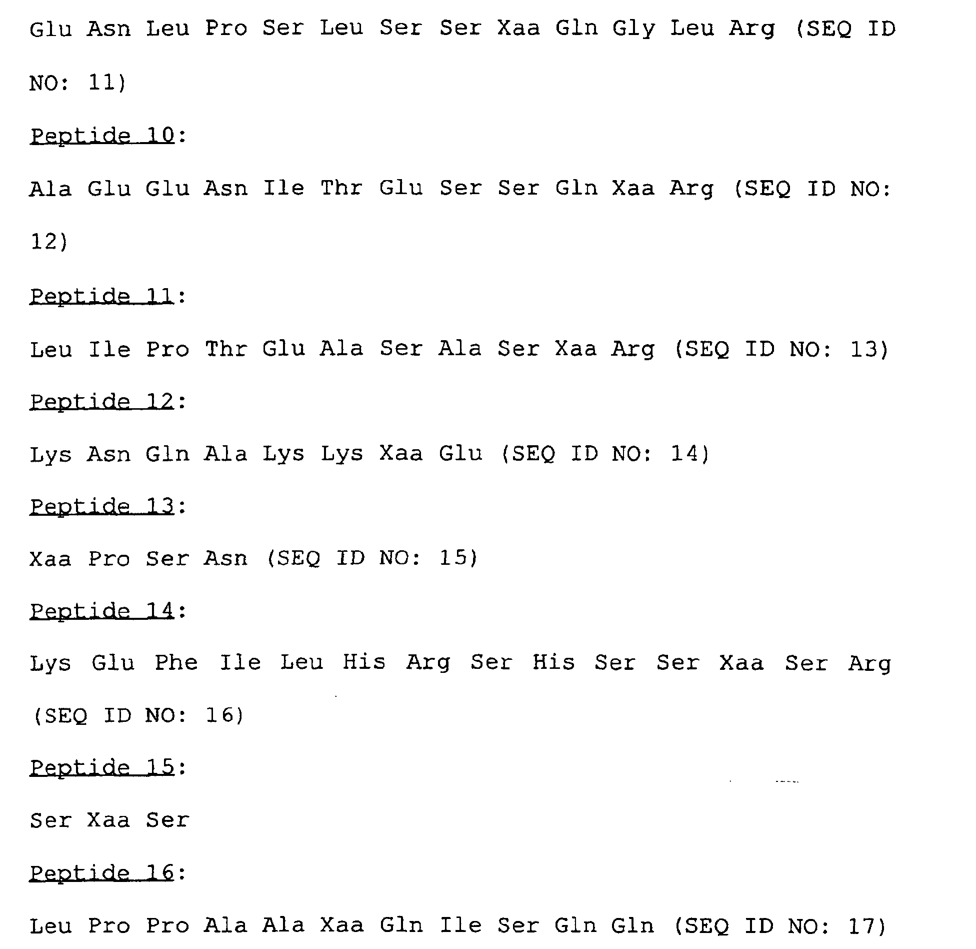

The amino acid sequence of peptide 14 corresponds to the amino acid sequence of peptide 6 in the direction from the C-terminal to the N-terminal thereof.

### Reference Example

### (Cytotoxicity-inhibitory Activity)

Using Dami cells (described in Greenberg S. M. et al., Blood, vol. 72, p. 1968-1977 (1988)) for use in assay system for cytotoxicity-inhibitory activity, the cells were washed twice with assay medium (50% PBS/SA/0.03% HSA (manufactured by SIGMA) or SA/0.05% BSA free from fatty acid (WAKO)/4 µM long-chain polyvalent fatty acid (e.g. arachidonic acid, linoleic acid or linolenic acid)) and suspended in the same medium at 3 × 10⁴ cells/ml. The cell suspension was added to a 96-well plate in each 200 µl for wells for sample addition or in each 100 µl for wells for serial dilution. To the wells for sample addition was added each 2 µl assay sample containing either peptides synthesized in Preparation 2, selenocystine, selenomethionine, Ebselen, or sodium selenite at the same concentration. After stirring, a serial dilution was made with the wells containing 100 µl cell suspension. The plate was incubated at 37°C in CO₂ incubator for 4 to 5 days followed by assessment of survival of the cells.

Cytotoxicity-inhibitory activity was determined for each of the peptides obtained in Preparation 2. As a result, selenocysteine-containing peptides, i.e. Peptide 3 through Peptide 16, were confirmed to have the cytotoxicity-inhibitory activity. Among these, Peptide 6, in which Cys residues were all replaced with Ser, could inhibit cytotoxicity most effectively at the lowest concentration. Also, no toxicity was observed for the selenocysteine-containing peptides even at a concentration where toxicity could be observed in other selenium-containing substances. See Table 2.

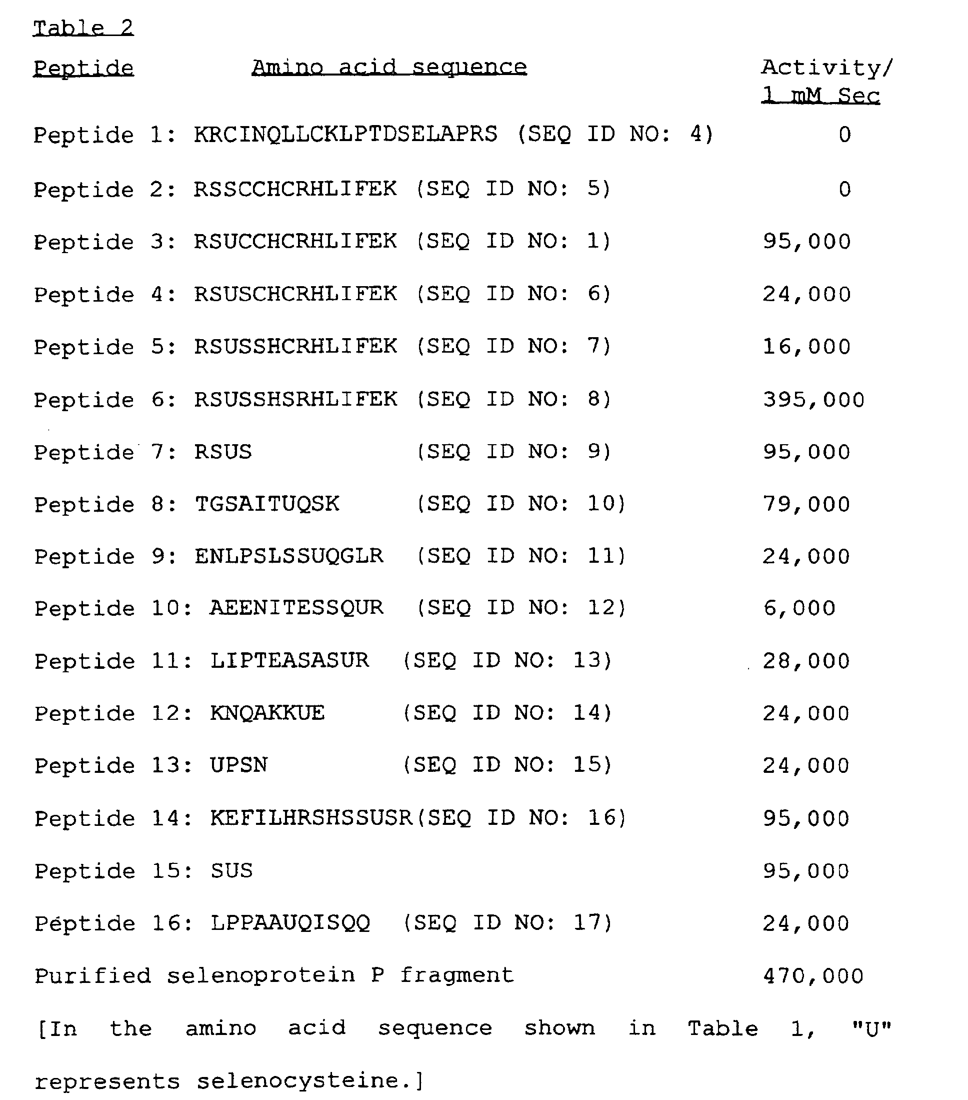

### Example 1

### (Effect of Selenoprotein P on Ataxia in Rolling Mouse Nagoya)

In order to confirm the activity to ameliorate ataxia of selenoprotein P, Rolling Mouse Nagoya, the established model of ataxia, was used with a stumbling index. Animals were divided into the following groups each consisting of ten animals: a control group intraperitoneally administered with saline (0.25 mL/head/week); a group intraperitoneally administered with a low dose of selenoprotein P (0.05 mg/head/week; 0.25 mL/head/week); and a group intraperitoneally administered with a high dose of selenoprotein P (0.5 mg/head/week; 0.25 mL/head/week). The animals received intraperitoneal administration once a week for three weeks.

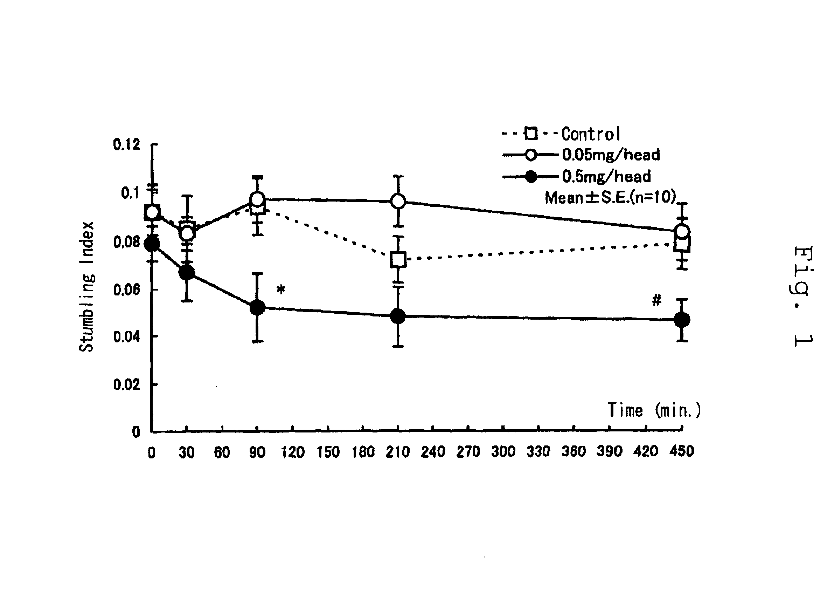

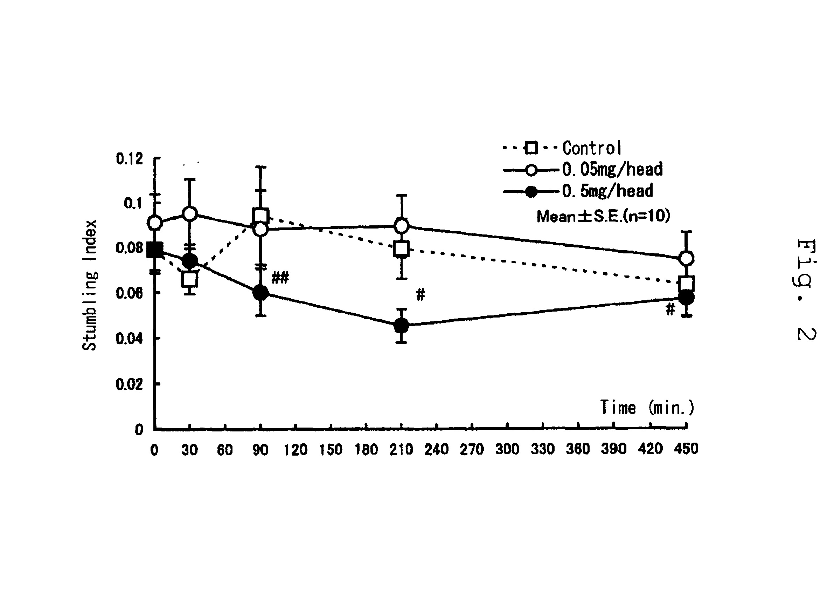

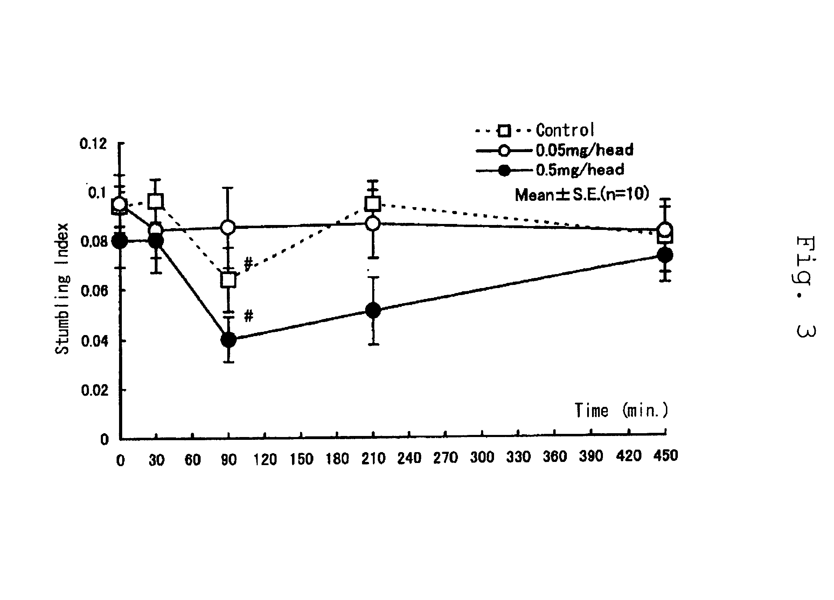

The animals were Rolling Mouse Nagoya (113 animals, five to eight months old, weighing 19.1 to 36.2 g) regardless of sex. Grouping of the animals was based on the weight measured on the day before initiation of the experiment and a stumbling index measured before initiation of the experiment. A quantity of voluntary motion was measured during thirty minutes immediately before administration (during 30-0 min. before administration), immediately after administration (during 0-30 min. after administration), one hour after administration (during 60-90 min. after administration), three hours after administration (during 180-210 min. after administration), and seven hours after administration (during 420-450 min. after administration) with a device for measuring voluntary motion. Stumbling of animals was counted with a video camera set upon the device for measuring voluntary motion during the same time period as that of measurement of voluntary motion. A stumbling index was calculated by the formula: [Count of stumbling (count/30 min.)] / [Voluntary motion (count/30 min.)]. The results are shown in Figs. 1 to 3 wherein #: p<0.05, ##: p<0.01, being significant as compared to values before administration (paired t-test); *: p<0.05, being significant as compared to control during the same time period (Turkey test).

It was demonstrated that a stumbling index in the group of a high dose administration of selenoprotein P (0.5 mg/head) remained lower than the control group at each time period on every Week 1, Week 2 and Week 3 after administration, with significant decrease during 60-90 min. after administration on Week 1. When compared with values before administration, significant decrease in a stumbling index was observed during 420-450 min. after administration on Week 1, during 60-90 min., during 180-210 min., and during 420-450 min. after administration on Week 2, and during 60-90 min. after administration on Week 3. On the contrary, no significant decrease in a stumbling index was seen in the group of low dose administration of selenoprotein P (0.05 mg/head).

Thus, the present invention provides with an excellent medicament for treating neurodegenerative diseases, especially suitable for treating neurodegenerative diseases with ataxia as a principal symptom.

## Claims

1. A medicament for treating neurodegenerative diseases, comprising as an active ingredient selenoprotein P and/or a peptide fragment or a series of peptide fragments derived from the C-terminal of selenoprotein P.

2. The medicament for treating neurodegenerative diseases according to claim 1 wherein said peptide fragment or a series of peptide fragments derived from the C-terminal of selenoprotein P is a peptide or a series of peptides with a cytotoxicity-inhibitory activity having the amino acid sequence from 260th to 362nd amino acid residues from the C-terminal of selenoprotein P, or said amino acid sequence with one or several amino acid residues therein being deleted, substituted or added, or a partial sequence of either of the above amino acid sequences, or an amino acid sequence comprising as a part any of the above amino acid sequences.

3. The medicament for treating neurodegenerative diseases according to claim 1 or 2 wherein said peptide fragment or a series of peptide fragments derived from the C-terminal of selenoprotein P is a peptide or a series of peptides having the amino acid sequences of the formula (I): Arg Ser Xaa Cys Cys His Cys Arg His Leu Ile Phe Glu Lys (SEQ ID NO: 1) wherein Xaa represents selenocysteine, or said amino acid sequences with one or several amino acid residues therein being deleted, substituted or added, or a partial sequence of either of the above amino acid sequences, or an amino acid sequence comprising as a part any of the above amino acid sequences.

4. The medicament for treating neurodegenerative diseases according to any one of claims 1 to 3 wherein the principal symptom due to neurodegenerative condition is ataxia.
